# EUROPÄISCHE PATENTSCHRIFT

(11) **EP 1 204 633 B1**
(45) Veröffentlichungstag und Bekanntmachung des Hinweises auf die Patenterteilung: **18.02.2004**
(21) Anmeldenummer: 00942114.0
(22) Anmeldetag: 21.06.2000
(51) Int. Cl.: C07C 277/08

(54) **VERFAHREN ZUR HERSTELLUNG VON 1-METHYL-3-NITROGUANIDIN**
METHOD FOR PRODUCING 1-METHYL-3-NITROGUANIDINE
PROCEDE DE PRODUCTION DE 1-METHYL-3-NITROGUANIDINE

(30) Priorität: 20.08.1999 DE 19939609
(43) Veröffentlichungstag der Anmeldung: 15.05.2002
(73) Patentinhaber: NIGU CHEMIE GMBH, D-84478 Waldkraiburg (DE)
(72) Erfinder: STENGELE, Klaus-Peter, D-84568 Pleiskirchen (DE); KERN, Norbert, D-84478 Waldkraiburg (DE); SCHULZ, Bernd, D-84478 Waldkraiburg (DE)
(74) Vertreter: HOFFMANN EITLE
(86) Internationale Anmeldenummer: PCT/EP2000/005757
(87) Internationale Veröffentlichungsnummer: WO 2001/014323

(56) Entgegenhaltungen:
- WO-A-98/43951
- US-A- 4 677 226
- US-A- 5 783 734
- LAWRENCE FISHBEIN AND JOHN A GALLAGHAN: "The preparation of 2-alkyl-1(or 3)-nitro-2-thiopseudourea" JOURNAL OF THE AMERICAN CHEMICAL SOCIETY., Bd. 76, 5. April 1954 (1954-04-05), Seiten 1877-79, XP002148829 AMERICAN CHEMICAL SOCIETY, WASHINGTON, DC., US ISSN: 0002-7863 in der Anmeldung erwähnt

## Beschreibung

Die vorliegende Erfindung betrifft ein Verfahren zur Herstellung von 1-Methyl-3-nitroguanidin. 1-Methyl-3-nitroguanidin stellt ein wichtiges Zwischenprodukt zur Herstellung von biologisch aktiven Verbindungen, insbesondere von Insektiziden dar (vgl. EP-A 376 279 bzw. EPA 428 941). Für die Herstellung von 1-Methyl-3-nitroguanidin sind schon eine Reihe von Verfahren entwickelt worden.

### Stand der Technik

So ist es bspw. bekannt, dass man 1-Methyl-3-nitroguanidin durch Nitrierung von Methylisothioharnstoffsulfat and anschließender Substitution der Methylmercaptogruppe gegen Methylamin erhalt (vgl. hierzu J. Amer. Chem. Soc. 1954, 76, 1877). Dabei wirft die Abspaltung von Methylmercaptan, vor allem bei einer Durchführung im großtechnischen Maßstab, erhebliche verfahrenstechnische Probleme auf.

Ferner wurde vorgeschlagen, 1-Methyl-3-nitroguanidin durch Umsetzung einer alkalischen Nitroguanidin-Lösung mit Methylammoniumchlorid bei 60 °C herzustellen (vgl. hierzu J. Amer. Chem. Soc. 1947, 69, 3028 and J. Amer. Chem. Soc. 1927, 49, 2303). Bei diesem Prozess sind die Ausbeuten and insbesondere die Reinheit der Endprodukte, die in aufwendigen Verfahren gereinigt werden müssen, sehr unbefriedigend. Als Nebenprodukte entstehen unter Entwicklung von gasförmigem N₂O größere Mengen Methylharnstoff and große Mengen an Abwasser. Nachteilig ist weiterhin, dass Nitroguanidin einen relativ teuren Ausgangsstoff darstellt.

Diese Nachteile betreffen auch die in der EP-A 798 293 beschriebene direkte Umsetzung von Methylamin mit Nitroguanidin. Auch bei dieser Vorgehensweise wird 1-Methyl-3-nitroguanidin nur in unbefriedigenden Ausbeuten erhalten. Außerdem wurde empfohlen, dass man 1-Alkyl-3-nitroguanidine durch Nitrierung von Alkylguanidinsulfaten erhalten kann (vgl. hierzu J. Amer. Chem. Soc. 1933, 55, 731). Auch diese Verfahrensvariante hat den Nachteil einer nicht zufriedenstellenden Ausbeute sowie einer relativ großen Abwassermenge.

Eine weitere bekannte Herstellungsmethode stellt die Dehydratisierung von Alkylguanidinnitraten mittels Schwefelsäure dar (vgl. hierzu J. Amer. Chem. Soc. 1933, 55,739). Das Alkylguanidinnitrat ist zwar gemäß US 2,425,341 aus wässriger Cyanamid-Lösung and Alkylaminnitrat zugänglich, doch ist das Verfahren wegen der relativ geringen Ausbeute and der erforderlichen Nachreinigung unbefriedigend.

Gemäß einer in WO 98/43 951 beschriebenen Variante dieses Weges wird Alkylguanidinnitrat durch Umsetzung von Alkylammoniumnitrat mit wasserfreiem Cyanamid in organischen Lösemitteln wie Ether oder Alkoholen erhalten and direkt danach zu 1-Alkyl-3-nitroguanidin umgesetzt. Die Verwendung wasserfreien Cyanamids ist jedoch im Vergleich zu wässriger Cyanamidlösung unwirtschaftlich.

Auch die Umsetzung von Methylammoniumnitrat mit Calciumcyanamid oder Dicyandiamid verläuft unbefriedigend (vgl. hierzu Can. J. Chem. 1958, 36, 737-743), da die Reinheit des so erhaltenen Methylguanidinnitrats weniger als 90% beträgt.

Gemäß dem US-Patent 4,677,226 wird die Herstellung von Alkyl, Alkenyl- oder Alkinyl-nitroguanidinen durch Umsetzung eines primären Amins mit einer N-Alkyl-N-nitroso-N'nitroguanidinverbindung in Gegenwart einer wässrigen alkoholischen Lösung bei etwa 40°C beschrieben. Auch dieses Verfahren erscheint technisch sehr aufwendig, da die entsprechende N-Alkyl-N-nitroso-N'nitroguanidinverbindung nur sehr schwierig herzustellen ist.

### Darstellung der Erfindung

Der vorliegenden Erfindung lag daher die Aufgabe zugrunde, ein Verfahren zur Herstellung von 1-Methyl-3-nitroguanidin zu entwickeln, welches die genannten Nachteile entsprechend dem Stand der Technik nicht aufweist, sondern ausgehend von kostengünstigen Ausgangsmaterialien technisch einfach durchführbar ist und hohe Ausbeuten und Reinheiten ermöglicht sowie nur geringe Mengen an Nebenprodukten verursacht.

Diese Aufgabe wurde erfindungsgemäß dadurch gelöst, dass man
a) Chlorcyan oder Bromcyan mit Methylamin in Gegenwart eines inerten organischen Lösemittels bei Temperaturen von - 30 bis 0 °C umsetzt, anschließend
b) das in Stufe a) gebildete Methylcyanamid ohne weitere Aufarbeitung direkt mit Ammoniumnitrat im Temperaturbereich von 80 bis 180°C und Druckbereich zwischen 1 und 50 bar reagieren lässt und
c) danach das in Stufe b) gebildete Methylguanidinnitrat nach Entfernen des Lösemittels bei Temperaturen von - 20 bis 60 °C dehydratisiert und
d) schließlich das in Stufe c) gebildete 1-Methyl-3-nitroguanidin isoliert.

Es hat sich hierbei überraschenderweise gezeigt, dass man 1-Methyl-3-nitroguanidin in sehr guten Ausbeuten und in hoher Reinheit erhält.

Dies war deshalb so überraschend, weil zu erwarten war dass die Trimerisierung von Methylcyanamid zu Trimethylisomelamin bevorzugt ablaufen sollte (vgl. hierzu J. Org. Chem. 1960, 25, 1043 - 1045).

Beim Verfahren entsprechend der vorliegenden Erfindung wird in der ersten Reaktionsstufe a) Chlorcyan oder Bromcyan mit Methylamin in Gegenwart eines inerten organischen Lösemittels bei Temperaturen von - 30 bis 0°C umgesetzt. Der Vorteil bei dieser Reaktionsstufe liegt darin, dass die Ausgangsverbindungen, insbesondere Chlorcyan, in großtechnischem Maßstab hergestellt werden und somit kostengünstige Ausgangsmaterialien darstellen.

Als organische Lösemittel kommen alle gegenüber den Reaktanden inerten organischen Verdünnungsmittel in Frage. Vorzugsweise gehören hierzu Ether wie Diethylether, Tetrahydrofuran, Ethylenglykoldimethylether, Ethylenglykoldiethylether, Diethylenglykoldimethylether oder Methyl-tert.butylether.

Das Molverhältnis von Chlorcyan oder Bromcyan zu Methylamin kann in weiten Grenzen variiert werden, es hat sich jedoch als besonders vorteilhaft erwiesen, das Molverhältnis von Chlorcyan oder Bromcyan zu Methylamin auf 1 : 1 bis 1 : 1,05 einzustellen.

Im Anschluss an die Reaktionsstufe a) wird das in Stufe a) gebildete Methylcyanamid in der Reaktionsstufe b) ohne weitere Aufarbeitung, vorzugsweise in demselben inerten Lösemittel wie in Stufe a), direkt mit Ammoniumnitrat im Temperaturbereich von 80 bis 180 °C, insbesondere zwischen 100 und 150 °C, und im Druckbereich zwischen 1 und 50 bar, insbesondere zwischen 4 und 20 bar, umgesetzt.

Gemäß einer bevorzugten Ausführungsform wird die Reaktionsstufe b) unter Sauerstoffausschluss durchgeführt und das Molverhältnis von Methylcyanamid : Ammoniumnitrat auf 1,5 : 1 bis 1 : 1,5 eingestellt.

Im Anschluss an die Reaktionsstufe b) wird das inerte organische Lösemittel nach bekannten Methoden, wie z. B. Destillation, abgetrennt und danach das in Stufe b) gebildete Methylguanidinnitrat in Reaktionsstufe c) bei Temperaturen von - 20 bis 60 °C, insbesondere bei - 10 bis 5 °C, dehydratisiert, wobei die Dehydratisierung vorzugsweise mit Hilfe von konzentrierter Schwefelsäure, Salpetersäure (92 - 98 %) oder Trifluoressigsäure durchgeführt wird.

Abschließend wird das in Stufe c) gebildete 1-Methyl-3-nitroguanidin in Stufe d) isoliert. Gemäß einer bevorzugten Ausführungsform (insbesondere bei Verwendung von konzentrierter Schwefelsäure) wird kaltes Wasser zugegeben und das Produkt vorzugsweise durch Filtration abgetrennt.

Im Rahmen der vorliegenden Erfindung ist es möglich, das Endprodukt mit etwas kaltem Wasser nachzuwaschen und ggf. zu trocknen.

Mit Hilfe des erfindungsgemäßen Verfahrens ist es möglich, 1-Methyl-3-nitroguanidin in Ausbeuten von mindestens 85 % und Reinheiten von > 98 % auf besonders umweltschonende und technisch einfache Weise zu erhalten.

Die nachfolgenden Beispiele sollen die Erfindung näher veranschaulichen.

### Beispiele

### Beispiel 1

21,2 g (0,20 mol) Bromcyan wird in 150 ml Tetrahydrofuran gelöst. Bei - 5 bis - 10 °C werden innerhalb von 1 h 12,4 g (0,4 mol) Methylamin eingeleitet. Nach Filtration des Methylammoniumbromids wird 16,0 g (0,2 mol) Ammoniumnitrat in die klare Lösung gegeben. Das Gemisch wird im Autoklav 8 h bei 130 °C gerührt. Nach Entfernung des Lösemittels wird bei - 5 bis 0 °C 50,0 g (0,5 mol) konz. Schwefelsäure vorsichtig zugegeben. Nach 30 Minuten werden 150 g Eis zugegeben, abfiltriert und mit wenig kaltem Wasser nachgewaschen. Man erhält 24,1 g (88,6 % der Theorie) 1-Methyl-3-nitroguanidin vom Schmelzpunkt 159 - 160 °C. Die Reinheit (HPLC) ist > 98%.

### Beispiel 2

61,5 g (0,80 mol) Chlorcyan wird bei - 10 °C in 600 ml Tetrahydrofuran eingeleitet, in dem 42,4 g (1,6 mol) Natriumcarbonat suspendiert sind. Bei - 5 bis - 10 °C werden innerhalb von 1 h 24,8 g (0,8 mol) Methylamin eingeleitet. Nach Filtration der Natriumsalze wird 64,0 g (0,8 mol) Ammoniumnitrat in die klare Lösung gegeben. Das Gemisch wird im Autoklav 8 h bei 130 °C gerührt. Nach Entfernung des Lösemittels wird bei - 5 bis 0 °C 200,0 g (2,0 mol) Schwefelsäure 98 % vorsichtig zugegeben. Nach 30 Minuten werden 600 g Eis zugegeben, abfiltriert und mit wenig kaltem Wasser nachgewaschen. Man erhält 93,6 g (86,0 % der Theorie) 1-Methyl-3-nitroguanidin vom Schmelzpunkt 159 - 160 °C. Die Reinheit (HPLC) ist > 98%.

## Patentansprüche

1. Verfahren zur Herstellung von 1-Methyl-3-nitroguanidin, **dadurch gekennzeichnet, dass** man
a) Chlorcyan oder Bromcyan mit Methylamin in Gegenwart eines inerten organischen Lösemittels bei Temperaturen von - 30 bis 0 °C umsetzt, anschließend
b) das in Stufe a) gebildete Methylcyanamid ohne weitere Aufarbeitung direkt mit Ammoniumnitrat im Temperaturbereich von 80 bis 180°C und Druckbereich zwischen 1 und 50 bar reagieren lässt und
c) danach das in Stufe b) gebildete Methylguanidinnitrat nach Entfernen des Lösemittels bei Temperaturen von - 20 bis 60 °C dehydratisiert und
d) schließlich das in Stufe c) gebildete 1-Methyl-3-nitroguanidin isoliert.

2. Verfahren nach Anspruch 1, **dadurch gekennzeichnet, dass** man als inertes organisches Lösemittel in Stufe a) und b) Ether, wie z. B. Diethylether, Tetrahydrofuran, Ethylenglykoldimethylether, Ethylenglykoldiethylether, Diethylenglykoldimethylether oder Methyl-tert.-butylether einsetzt.

3. Verfahren nach den Ansprüchen 1 und 2, **dadurch gekennzeichnet, dass** man das Molverhältnis von Chlorcyan oder Bromcyan zu Methylamin in Stufe a) auf 1 : 1 bis 1 : 1,05 einstellt.

4. Verfahren nach den Ansprüchen 1 bis 3, **dadurch gekennzeichnet, dass** man die Reaktionsstufe b) unter Sauerstoffausschluss durchführt.

5. Verfahren nach den Ansprüchen 1 bis 4, **dadurch gekennzeichnet, dass** man die Reaktionsstufe b) im Temperaturbereich von 100 bis 150 °C und Druckbereich von 4 bis 20 bar durchführt.

6. Verfahren nach den Ansprüchen 1 bis 5, **dadurch gekennzeichnet, dass** das Molverhältnis von Methylcyanamid zu Ammoniumnitrat auf 1,5 : 1 bis 1 : 1,5 eingestellt wird.

7. Verfahren nach den Ansprüchen 1 bis 6, **dadurch gekennzeichnet, dass** die Dehydratisierung in Stufe c) mit Hilfe von konzentrierter Schwefelsäure, Salpetersäure oder Trifluoressigsäure erfolgt.

8. Verfahren nach den Ansprüchen 1 bis 7, **dadurch gekennzeichnet, dass** man Stufe c) bei einer Temperatur von - 10 bis 5 °C durchführt.

## Claims

1. Process for producing 1-methyl-3-nitroguanidine, **characterised in that**
a) cyanogen chloride or cyanogen bromide is reacted with methylamine in the presence of an inert organic solvent at temperatures from -30 to 0°C, then
b) the methylcyanamide formed in stage a) is allowed to react directly with ammonium nitrate without further working up in the temperature range from 80 to 180°C and pressure range between 1 and 50 bar and
c) then the methylguanidine nitrate formed in stage b) is dehydrated after removing the solvent at temperatures from -20 to 60°C and
d) finally the 1-methyl-3-nitroguanidine formed in stage c) is isolated.

2. Process according to claim 1, **characterised in that** ether, such as for example diethyl ether, tetrahydrofuran, ethylene glycol dimethyl ether, ethylene glycol diethyl ether, diethylene glycol dimethyl ether or methyl-tert.-butyl ether is used as inert organic solvent in stage a) and b).

3. Process according to claims 1 and 2, **characterised in that** the molar ratio of cyanogen chloride or cyanogen bromide to methylamine in stage a) is set at 1:1 to 1:1.05.

4. Process according to claims 1 to 3, **characterised in that** the reaction stage b) is carried out with exclusion of oxygen.

5. Process according to claims 1 to 4, **characterised in that** the reaction stage b) is carried out in the temperature range from 100 to 150°C and pressure range from 4 to 20 bar.

6. Process according to claims 1 to 5, **characterised in that** the molar ratio of methylcyanamide to ammonium nitrate is set at 1.5:1 to 1:1.5.

7. Process according to claims 1 to 6, **characterised in that** the dehydration in stage c) is effected with the aid of concentrated sulphuric acid, nitric acid or trifluoroacetic acid.

8. Process according to claims 1 to 7, **characterised in that** stage c) is carried out at a temperature from -10 to 5°C.

## Revendications

1. Procédé de production de 1-méthyl-3-nitroguanidine **caractérisé en ce qu'**
a) on convertit du chlorcyane ou du bromocyane avec de la méthylamine en présence d'un solvant organique inerte, à des températures de -30 à 0°C, puis
b) on laisse réagir le méthylcyanamide formé à l'étape a) sans autre préparation, directement avec du nitrate d'ammonium, dans une plage de températures de 80 à 180°C et dans une plage de pressions comprise entre 1 et 50 bar, et
c) ensuite, après élimination du solvant, à des températures de -20 à 60°C, on déshydrate le nitrate de méthylguanidine ayant été formé à l'étape b), et
d) enfin, on isole la 1-méthyl-3-nitroguanidine formée à l'étape c).

2. Procédé selon la revendication 1, **caractérisé en ce qu'**on utilise comme solvant organique inerte aux étapes a) et b), éther, tel que par exemple un diéthyléther, tétrahydrofurane, éthylèneglycoldiméthyléther, éthylèneglycoldiéthyléther, diéthylèneglycoldiméthyléther ou méthyl-tert.-butyléther.

3. Procédé selon les revendications 1 et 2, **caractérisé en ce qu'**on règle la rapport molaire du chlorcyane ou du bromocyane par rapport à la méthylamine à l'étape a) à une valeur de 1 : 1 à 1 : 1,05.

4. Procédé selon les revendications 1 à 3, **caractérisé en ce que** l'on effectue l'étape de réaction b) sous exclusion d'oxygène.

5. Procédé selon les revendications 1 à 4, **caractérisé en ce que** l'on effectue l'étape de réaction b) dans une plage de températures de 100 à 150°C et dans une plage de pressions de 4 à 20 bar.

6. Procédé selon les revendications 1 à 5, **caractérisé en ce que** le rapport molaire entre méthylcyanamide et nitrate d'ammonium est réglé à 1,5 : 1 jusqu'à 1 : 1,5.

7. Procédé selon les revendications 1 à 6, **caractérisé en ce que** l'on effectue la déshydratation à l'étape c) à l'aide d'acide sulfurique, d'acide nitrique ou d'acide trifluoroacétique, concentré.

8. Procédé selon les revendications 1 à 7, **caractérisé en ce que** l'on effectue l'étape c) à une température comprise dans la plage de -10 à 5°C.
